# EUROPEAN PATENT SPECIFICATION

(11) **EP 2 846 862 B1**
(45) Date of publication and mention of the grant of the patent: **25.10.2017**
(21) Application number: 13735067.4
(22) Date of filing: 10.05.2013
(51) Int. Cl.: A61M 16/06

(54) **PATIENT INTERFACE DEVICE INCLUDING DEFORMABLE NASAL PRONG**
PATIENTENSCHNITTSTELLENVORRICHTUNG MIT VERFORMBARER NASENGABEL
DISPOSITIF D'INTERFACE PATIENT COMPRENANT UNE PINCE NASALE DÉFORMABLE

(30) Priority: 10.05.2012 US 201261645215 P
(43) Date of publication of application: 18.03.2015
(73) Proprietor: Koninklijke Philips N.V., 5656 AE Eindhoven (NL)
(72) Inventor: FRANZEN, Erika, 5656 AE Eindhoven (NL)
(74) Representative: Steffen, Thomas
(86) International application number: PCT/IB2013/053798
(87) International publication number: WO 2013/168134

(56) References cited:
- WO-A1-2009/151344
- WO-A2-2011/086438
- US-A- 4 782 832
- US-A1- 2005 166 927
- US-A1- 2008 289 633

## Description

### BACKGROUND OF THE INVENTION

### 1. Field of the Invention

The present invention relates to patient interface devices for transporting a gas to and/or from an airway of a patient and, in particular, to a patient interface device including a pillows-style nasal cushion having deformable nasal prongs, at least a portion of which are received in the nostrils of the patient and are engaged therewith.

### 2. Description of the Related Art

Numerous situations exist wherein it is necessary or desirable to deliver a flow of breathing gas non-invasively to an airway of a patient, i.e., without intubating the patient or surgically inserting a tracheal tube in the esophagus. For example, it is known to ventilate a patient using a technique known as non-invasive ventilation. It is also known to deliver continuous positive airway pressure (CPAP) or variable airway pressure, which varies with the patient's respiratory cycle, to treat a medical disorder such as sleep apnea syndrome, in particular, obstructive sleep apnea (OSA), or congestive heart failure.

Non-invasive ventilation and pressure support therapies involve the placement of a patient interface device including a mask component on the face of a patient. The mask component may be, without limitation, a nasal mask that covers the patient's nose, a nasal cushion that rests beneath the patient's nose (such as a "pillows" style nasal cushion having nasal prongs that are received within the patient's nostrils or a "cradle" style nasal cushion that rests beneath and covers the patient's nostrils), a nasal/oral mask that covers the nose and mouth, or a full face mask that covers the patient's face. The patient interface device interfaces the ventilator or pressure support device with the airway of the patient so that a flow of breathing gas can be delivered from the pressure/flow generating device to the airway of the patient. It is known to maintain such devices on the face of a wearer by a headgear having one or more straps adapted to fit over/around the patient's head.

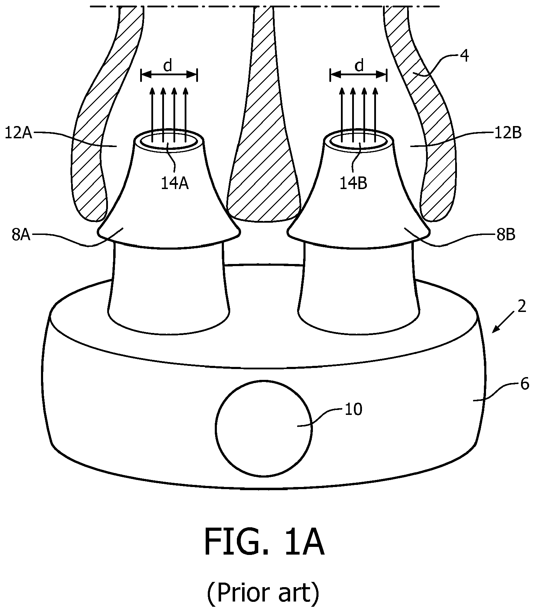

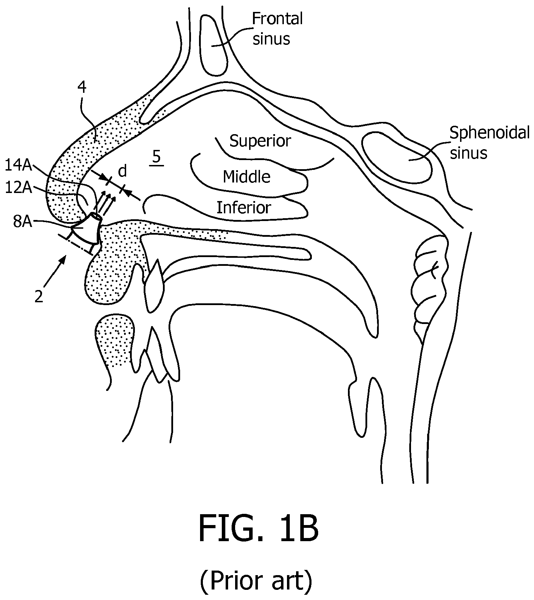

FIGS. 1A and 1B are schematic diagrams showing a prior art pillows style nasal cushion 2 inserted into the nose 4 (shown in cross-section) of a patient. FIG. 1B shows a side view of the entire nasal passage 5 of the patient. Nasal cushion 2 is made of a flexible, cushiony, elastomeric material, such as, without limitation, silicone, an appropriately soft thermoplastic elastomer, a closed cell foam, or any combination of such materials, and includes a main body portion 6 having nasal prongs 8A and 8B extending from a top side thereof and an orifice 10 structured to be fluidly coupled to a fluid coupling conduit (not shown), such as an elbow connector, that is coupled to a ventilator or pressure support device. Each nasal prong 8A, 8B is structured to be received within a respective nostrils 12A, 12B of the patient and has an orifice 14A, 14B having a fixed diameter (d) through which the flow of breathing gas is delivered from inside nasal cushion 2 to the nostrils 12A, 12B of the patient.

US 4,782,832 A discloses a nasal puff assembly including a plenum chamber having an inlet and a pair of laterally spaced outlets, wherein a pair of soft synthetic resin nare elements or pillows are operatively coupled with the spaced plenum outlets and are designed for ready adjustability so that the effective positions thereof can be altered individually for permitting custom fitting after nare elements with respect to the nares of different patients.

A nasal prong, which forms the basis for the preamble of independent claim 1, is known from WO 2011/086438 A2.

Further exemplary nasal prongs and pillows are known from US 2008/0289633 A1 and from WO 2009/151344 A1.

### SUMMARY OF THE INVENTION

Accordingly, it is an object of the present invention to provide a patient interface device and a prong that overcomes the shortcomings of conventional patient interface devices.

This object is achieved according to one embodiment of the present invention by providing a nasal prong according to claim 1 that is structured to be situated on a nasal cushion of a patient interface device that is in fluid communication with a source of breathing gas and that is structured to provide a flow of breathing gas to an airway of a patient. The nasal prong includes a resilient seal apparatus which comprises a deformable element and a fixable enclosure that possesses elastic and resilient properties, at least a portion of the deformable element being situated within the flexible enclosure, wherein the resilient seal apparatus is structured to be deformable between a free state and a deformed state from among a plurality of deformed states in response to a force applied to the seal apparatus and being further structured to return from the deformed state in a direction generally toward the free state upon removal of the force. At least a portion of the seal apparatus that is in response to compressive forces applied to the seal apparatus in a first deformed state and that is received in a nostril of the patient is structured to move toward the free state and to compressively and sealingly engage the nostril in a second deformed state that is generally between the first deformed state and the free state. The nasal prong further includes a flow channel extending through the seal apparatus, the flow channel being structured to be in fluid communication with the source of breathing gas and to provide the flow of breathing gas into the nostril. The deformable element of the resilient seal apparatus comprises a quantity of fluid in a liquid state and being retained within the enclosure.

The enclosure includes a first portion and a second portion that are formed out of the same material. The first portion has a relatively greater stiffness than the second portion.

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention.

### BRIEF DESCRIPTION OF THE DRAWINGS

FIGS. 1A and 1B are schematic diagrams showing a known pillow style nasal cushion inserted into the nose of a patient;
FIG. 2 is a diagrammatic depiction of a first embodiment of an improved nasal prong of an improved patient interface device in proximity to a nostril of a patient, the nasal prong being in a first deformed state;
FIG. 3 is another depiction of the nasal prong of FIG. 2, the nasal prong being in a free state;
FIG. 4 is a diagrammatic depiction of the nasal prong of FIG. 2, at least a portion of the nasal prong being received inside the nostril and being in a second deformed state engaged with the nostril;
FIG. 5 is a sectional view as taken along line 5-5 of FIG. 3;
FIG. 6 is a view similar to FIG. 5, except depicting an improved nasal prong in accordance with a second embodiment of the disclosed and claimed concept;
FIGS. 7A and 7B are views of an improved nasal prong in accordance with a third embodiment of the disclosed and claimed concept in a free state;
FIGS. 8A and 8B are views of the nasal prong of FIGS. 7A and 7B, except in a first deformed state;
FIGS. 9A and 9B are views of the nasal prong of FIGS. 7A and 7B in a second deformed state partially returned to the free state from the first deformed state;
FIGS. 10A-C are depictions of the profiles of three other nasal prongs in accordance with three additional embodiments of the disclosed and claimed concept;
FIGS. 11A-C are depictions of the form factors of three other nasal prongs in accordance with three further embodiments of the disclosed and claimed concept; and
FIGS. 12A and 12B depict the nasal prong of FIG. 11A and further depict another nasal prong in accordance with a tenth embodiment of the disclosed and claimed concept.

### DETAILED DESCRIPTION OF EXEMPLARY EMBODIMENTS

As used herein, the singular form of "a", "an", and "the" include plural references unless the context clearly dictates otherwise. As used herein, the statement that two or more parts or components are "coupled" shall mean that the parts are joined or operate together either directly or indirectly, i.e., through one or more intermediate parts or components, so long as a link occurs. As used herein, "directly coupled" means that two elements are directly in contact with each other. As used herein, "fixedly coupled" or "fixed" means that two components are coupled so as to move as one while maintaining a constant orientation relative to each other.

As used herein, the word "unitary" means a component is created as a single piece or unit. That is, a component that includes pieces that are created separately and then coupled together as a unit is not a "unitary" component or body. As employed herein, the statement that two or more parts or components "engage" one another shall mean that the parts exert a force against one another either directly or through one or more intermediate parts or components. As employed herein, the term "number" shall mean one or an integer greater than one (i.e., a plurality).

Directional phrases used herein, such as, for example and without limitation, top, bottom, left, right, upper, lower, front, back, and derivatives thereof, relate to the orientation of the elements shown in the drawings and are not limiting upon the claims unless expressly recited therein.

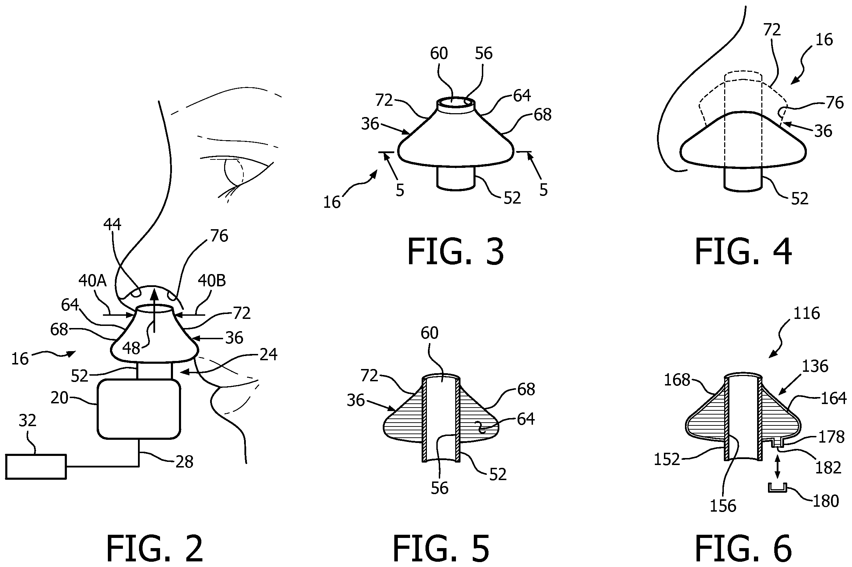

An improved nasal prong 16 in accordance with the first embodiment of the disclosed and claimed concept is depicted generally in FIGS. 2-3. Nasal prong 16 is depicted in FIG. 2 as being mounted on a nasal cushion 20 of an improved patient interface device 24 that is likewise in accordance with the disclosed and claimed concept. Patient interface device 24 is depicted as having a schematic connection 28 with a schematic source of breathing gas 32 such as a CPAP machine or other source of breathing gas.

Nasal prong 16 can be said to include a resilient seal apparatus 36 which is deformable between a free state, such as is depicted generally in FIG. 3, and any of a plurality of deformed states, one of which is depicted generally in FIG. 2 and another of which is depicted generally in FIG. 4. As employed herein, the expression "resilient" and variations thereof shall refer generally to the possession of a property whereby a body that has been changed via deformation from a free state to a deformed state will return from the deformed state in a direction generally toward the free state whenever the forces that caused the deformation are removed from the body.

Depending upon the direction and magnitude of the forces applied to seal apparatus 36, seal apparatus 36 can be in any of a wide variety of deformed states. By way of example, nasal prong 16 is depicted in FIG. 3 as being in a free state. In contrast, nasal prong 16 is depicted in FIG. 2 as being in a first deformed state as a result of being subjected to a pair of opposing compressive forces as indicated at the numerals 40A and 40B. It can be seen from FIG. 2 that the transverse dimension of seal apparatus 36 has been reduced, i.e., narrowed, as a result of the compressive forces 40A and 40B. By way of further contrast, nasal prong 16 is depicted in a second deformed state in FIG. 4, which is intended to demonstrate a deformed state of nasal prong that is partially returned to the free state of FIG. 3 from the first deformed state of FIG. 2. That is, seal apparatus 36 has expanded at least partially along at least a portion of its transverse dimension as compared with the first deformed state of FIG. 2.

In taking advantage of the benefits provided by nasal prong 16, nasal prong 16 is desirably subjected to forces such as compressive forces 40A and 40B, which will deform seal apparatus 36 sufficiently that it can be received in a nostril 44 of a patient as is indicated generally with the insertion arrow 48 in FIG. 2. When a deformed portion of seal apparatus 36 is received within the interior of nostril 44, and when the deformation forces such as compressive forces 40A and 40B are removed therefrom, the resilient properties of seal apparatus 36 cause seal apparatus 36 to tend to resiliently return from its first compressed state in a direction generally toward the free state.

However, when a portion of seal apparatus 36 is received in the interior of nostril 44, the resilient return of seal apparatus 36 toward its free state will cause such portion of seal apparatus 36 to become compressively and sealingly engaged with an inner surface 76 of the nostril 44, as is indicated generally in FIG. 4. That is, the portion of seal apparatus 36 that is received in nostril 44 will be resisted from fully return to its free state since the inner surface 76 of nostril 44 will interfere with such a complete return to the free state. Rather the resilient forces of the portion of seal apparatus 36 received in nostril 44 will cause that portion of seal apparatus 36 to expand into compressive and sealing engagement with the inner surface 76 of nostril 44, as is indicated generally in FIG. 4, and which can said to be a second deformed state of seal apparatus 36.

Compressive forces 40A and 40B can be said to deform seal apparatus 36 from its free state (as is indicated in FIG. 3) to a first deformed state (as is indicated in FIG. 2). Upon removal of compressive forces 40A and 40B, the resilient properties of seal apparatus 36 would tend to eventually return nasal prong 16 to its free state if nasal prong 16 were situated in a location where such return would be entirely unobstructed. However, since in FIG. 4, a portion of seal apparatus 36 is received inside nostril 44, nostril 44 will interfere with the complete return of seal apparatus 36 from the first deformed state of FIG. 2 to the free state of FIG. 3, and rather seal apparatus 36 will ultimately reach a second deformed state (FIG. 4). The second deformed state represents a partial return of the seal apparatus 36 from its first deformed state to its free state, in which depicted exemplary condition seal apparatus 36 is compressively and sealingly engaged with the inner surface 76 of nostril 44.

As can be understood from FIGS. 2-5, nasal prong 16 further includes a flow element 52, which is an elongated element having a flow channel 56 formed therein substantially throughout its longitudinal extent. Flow channel 56 is in fluid communication with nasal cushion 20 and thus is likewise also in fluid communication via connection 28 with source of breathing gas 32. Breathing gas is supplied via flow channel 56 and flows out of an exit 60 at a distal end of flow element 52 and into nostril 44 to provide the flow of breathing gas to the patient. Flow element 52 can be any of a wide variety of structures and, in the depicted exemplary embodiment, is a flexible tube element made from a resilient material such as a silicone rubber or other appropriate material.

As can be understood from FIG. 5, seal apparatus 36 can be said to include a deformable element 64 that is situated adjacent flow element 52, and seal apparatus 36 can further be said to include an enclosure 68 that at least partially contains therein deformable element 64. Enclosure 68 can be said to have an outer surface 72 which, when received in nostril 44 as set forth above, becomes compressively and sealingly engaged with the inner surface 76 of nostril 44.

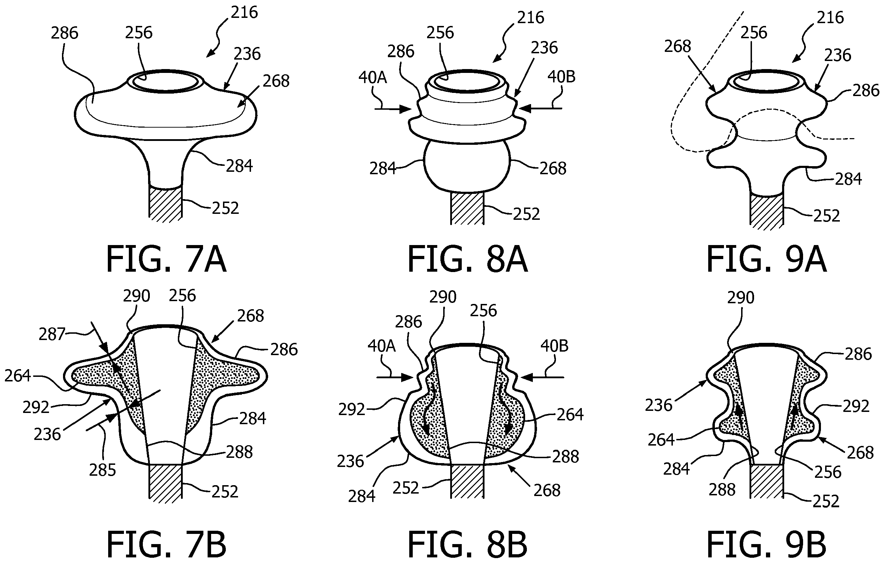

Deformable element 64 can be formed from any of a wide variety of materials such as closed cell foam, open cell foam, syntactic foam, or other deformable and/or resilient materials. In an embodiment in which deformable element 64 is itself resilient in addition to being deformable, enclosure 68 can be formed from materials having any of a wide variety of properties and is desirably at least flexible though it would need not necessarily be elastic. On the other hand, and as will be set forth in greater detail below in the context of FIGS. 7A-9B, if the deformable element is deformable but not necessarily resilient, such as a liquid material, the enclosure will desirably be elastic or resilient, by way of example.

In the exemplary embodiment depicted generally in FIGS. 2-5, deformable element 64 is a closed cell foam, and enclosure 68 is an integral skin that is integral to the foam of deformable element 64. That is, deformable element 64 is made out of a material, and outer surface 72 is formed at least partially of the same material, with deformable element 64 and enclosure 68 being co-formed as a single piece unit. Moreover, deformable element 64 and enclosure 68 may be co-formed in situ about the exterior of flow element 52 to form nasal prong 16. The material from which deformable element 64 is formed is configured to have specific resilient properties that enable the material to be compressible in a predetermined fashion, to be deformable in a predetermined fashion, and to be returnable in a predetermined fashion in a direction generally toward its free state.

Patient interface 24 most typically includes a pair of nasal prongs 16 on nasal cushion 20. In order to install patient interface device 24 on the patient, nasal prongs 16 are subjected to forces such as compressive forces 40A and 40B to deform seal apparatus 36 sufficiently that at least a portion of seal apparatus 36 can be received within the interior of nostril 44. Upon such insertion, compressive forces 40A and 40B are no longer being applied to nasal prongs 16, and the seal apparatus 36 of each nasal prong 16 is thus permitted to resiliently return in a direction generally toward the free state of seal apparatus 36. However, since at least a portion of each nasal prong 16 and, more particularly, seal apparatus 36 thereof, has been received within the interior of each nostril 44 of the patient, such resilient return of seal apparatus 36 toward the free state will continue until seal apparatus 36 compressively and sealingly engages inner surface 76 of nostril 44, which can be considered to be a second deformed state of nasal prong 16. The second deformed state can be generally said to be a state or degree of deformation that is between the first deformed state of nasal prong 16, such as that which enabled nasal prong 16 to be at least partially received in nostril 44, and the free state of nasal prong 16. The compressive and sealing engagement of outer surface 72 of seal apparatus 36 against inner surface 76 of nostril 44 retains nasal prong 16 in its position received in nostril 44 and thus at least partially retains patient interface device 24 on the patient.

That is, the compressive engagement between seal apparatus 36 and nostril 44 provides at least a portion of the retention forces that are required to retain patient interface device 24 in a given position with respect to the patient, thereby at least in part obviating the need for an extensive headgear, straps, and the like. It may be desirable to provide at least a simple headgear or a strap of some type to provide some supplemental retention forces to avoid unintentional disengagement of either nasal prong 16 from the nostrils 44 of the patient, although such headgear could be made optional and potentially may be unnecessary depending upon the configuration of nasal prong 16. Moreover, the compressive and sealing engagement of seal apparatus 36 within nostril 44 provides a seal between nasal prong 16 and nostril 44 that is substantially fluid-tight within the range of pressures that are typically expected to be experienced by patient interface device 24, which enhances the likelihood that the therapeutic flow of breathing gas provided by source of breathing gas 32 will actually be therapeutically provided to the airways of the patient.

An improved nasal prong 116 in accordance with a second embodiment of the disclosed and claimed concept is depicted generally in section in FIG. 6. That is, FIG. 6 is a view similar to FIG. 5, except that FIG. 6 depicts a second embodiment of an improved nasal prong in accordance with the invention in a free state.

Nasal prong 116 is similar to nasal prong 16 and includes a resilient seal apparatus 136 situated about a flow element 152 having a flow channel 156 formed therein. It also includes a deformable element 164 situated within an enclosure 168. However, the deformable element 164 is, in the embodiment depicted generally in FIG. 6, formed of an open cell foam, and enclosure 168 is in the form of a bladder that substantially contains deformable element 164 therein. Enclosure 168 is configured to provide for communication of air between deformable element 164 and the atmosphere, and thus is depicted herein in an exemplary fashion as being formed to further have a nipple 178 that extends therefrom and upon which is mounted a removable cap 180. Nipple 178 has an opening 182 formed therein that provides fluid communication between the interior of enclosure 168 and the atmosphere, which enables deformable element 164 to be compressed or otherwise deformed by enabling at least some of the air that is disposed or entrained within, say, the open cells of deformable element 164 to flow through opening 182. The flow of air through opening 182 can be resisted by receiving cap 180 on nipple 178, thereby retaining deformable element 168 in, by way of example, a deformed state, until cap 180 is removed from nipple 178. For instance, cap 180 can be removed from nipple 178, and deformable element 184 can be compressively deformed, which causes air to flow out of enclosure 168 and into the atmosphere through opening 182. Cap 180 can then be installed on nipple 178, which resists the resilient properties of deformable element 36 from returning to its free state since the flow of air into opening 82 and into enclosure 168 is resisted by the presence of cap 180. The presence of the cap 180 thus retains deformable element 164 in, for example, its first deformed state.

With at least a portion of seal apparatus 136 received in the nostril 44 of the patient, however, cap 180 can be removed, thereby permitting air to flow through opening 182 and into the enclosure 168, permitting the resilient properties of deformable element 164 to return it in a direction generally toward its free state. Since a portion of seal apparatus 136 is received in nostril 44, that portion of seal apparatus 136 will engage nostril 44 in a compressive and sealing fashion in a second deformed state of seal apparatus 136. The first and second deformed states of nasal prong 116 are similar to those of nasal prong 16.

An improved nasal prong 216 in accordance with a third embodiment of the disclosed and claimed concept is indicated generally in FIGS. 7A-9B. Nasal prong 216 includes a resilient seal apparatus 236 that is situated about a flow element 252 having a flow channel 256 formed therein. However, seal apparatus 236 includes a deformable element 264 that includes a quantity of fluid which, depending upon the particular properties of the embodiment, may be in a liquid state of matter and which will have various predetermined viscous properties. Deformable element 264 thus is retained within an enclosure 268 that possesses elastic and resilient properties.

In the depicted exemplary embodiment, enclosure 268 includes a first portion 284 and second portion 286 that are formed out of the same material. However, first portion 284 has a relatively greater stiffness in bending than second portion 286. In the depicted exemplary embodiment, such relatively greater stiffness of first portion 284 is the result of first portion 284 having a first thickness 285 that is relatively greater than a second thickness 287 of second portion 286. Thus, while in the depicted exemplary embodiment enclosure 268 (and thus first portion 284 and second portion 286) is formed of a single material, the relatively greater thickness at 285 than at 287 affords to first portion 284 a relatively greater stiffness and resistance to deformation such as bending than that afforded by second portion 286.

First portion 284 has a connection 288 with flow element 252, and second portion 286 likewise has a connection 290 with flow element 252. First and second portions 284 and 286 are additionally connected with one another at a further connection 292. The quantity of fluid that makes up deformable element 264 thus is situated generally between flow element 252 and first and second portions 284 and 286.

A free state of nasal prong 216 is depicted generally in FIGS. 7A and 7B. However, upon the application of forces to second portion 286, the quantity of fluid of deformable element 264 is caused to move, i.e., deform, which causes deformation of enclosure 268. That is, forces applied to second portion 286 cause the second portion 286 and deformable element 264 to be deformed. If forces such as compressive forces 40A and 40B are applied to second portion 286, as is indicated generally in FIGS. 8A and 8B, second portion 286 is deformed generally radially inwardly toward flow element 252. Such deformation of second portion 286 and the resultant deformation of deformable element 264 causes first portion 284 to deform in a direction generally outward and away from flow element 252 to cause nasal prong 216 to be in a first deformed state.

Upon deforming nasal prong 216 sufficiently that it can be received in nostril 44, compressive forces 40A and 40B are removed from nasal prong 216, and seal apparatus 236 is permitted to return in a direction generally toward its free state. Such return in a direction generally toward the free state is driven at least in part by the elastic properties of enclosure 268 and, in particular, by the relatively greater stiffness of first portion 284 in tending to return with relatively greater elastic force toward its free state than the elastic force of second portion 286 tending to return it to its own free state. Whereas first portion 284 is depicted in the first deformed state of FIGS. 8A and 8B as being bulged in an outward direction away from flow element 252, the removal of compressive forces 40A and 40B permits the elastic properties of first portion 284 to elastically return at least a portion of first portion 284 towards its free state, which results in movement of at least a portion of the quantity of fluid of deformable element 264 in a direction generally outwardly to deform second portion 286 until it compressively engages nostril 44. Such a second deformed state is indicated generally in FIGS. 9A and 9B. It thus can be seen that the resilient properties of seal apparatus 236, particularly including the elastic properties of enclosure 268 mentioned above, enable nasal prong 216 to return in a direction generally toward its free state from any of its various deformed states. It is noted, of course, that such return to the free state will be impeded for any portion of nasal prong 216 that is received in nostril 44 and that thus becomes compressively engaged therewith, as in the aforementioned second deformed state of nasal prong 216.

It is noted that any of a variety of materials can be employed in forming any of seal apparatuses 36, 136, and 236, it being expressly noted that the resilient properties can be provided by the deformable element, the enclosure, or a combination of the two. Moreover, the deformation properties and the properties whereby the seal apparatus 36, 136, and 236 returns in a direction generally toward its free state (but not necessarily reaching its free state when in, say, the second deformed state) can be predetermined based upon various properties of the seal apparatus, such as the material properties of its components, its shapes, configurations, thicknesses, other mechanical features, and the like without limitation.

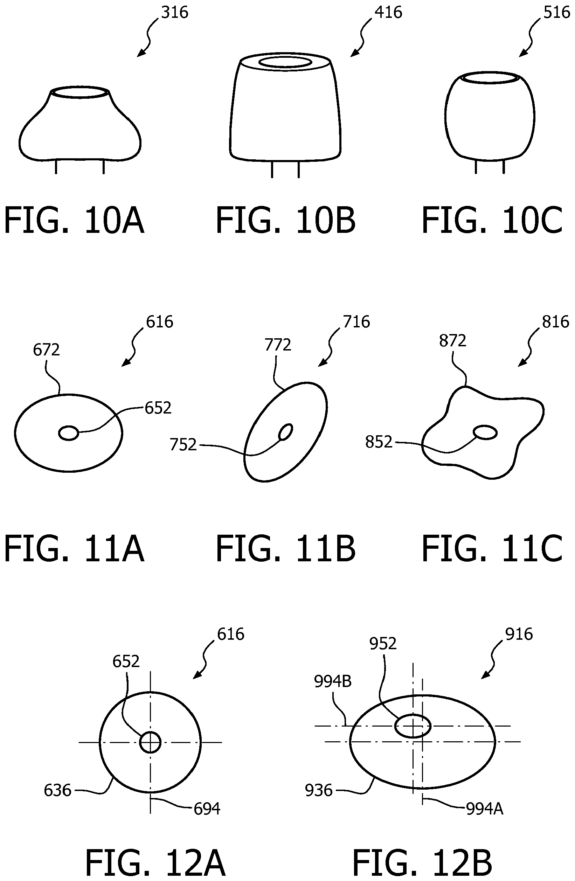

For example, and as can be seen from FIGS. 10A, 10B, and 10C which depict nasal prongs 316, 416, and 516 in accordance with fourth, fifth, and sixth embodiments of the disclosed and claimed concept, the profile of the nasal prong can be in any of a wide variety of shapes without departing from the present concept. For example, nasal prong 316 has a profile that is of a generally conic configuration, as is indicated generally in FIG. 10A, and which is similar to nasal prongs 16 and 116. As an alternative, nasal prong 416 is of a generally cylindrical profile, as is indicated generally in FIG. 10B. Likewise, nasal prong 516 is of a generally spherical profile as is indicated generally in FIG. 10C. Other variations will be apparent to one of ordinary skill in the art. It thus is to be understood that the specific shapes and profiles that are depicted herein are not intended to be limiting.

Further examples of nasal prongs are depicted generally in FIGS. 11A, 11B, and 11C, which depict nasal prongs 616, 716, and 816 respectively, in accordance with seventh, eighth, and ninth embodiments of the disclosed and claimed concept. FIGS. 11A-11C can be said to be top-down plan views of nasal prongs 616, 716, and 816, and can be said to depict the form factor of each. For example, the form factor of nasal prongs 616 is that of a fixed radius, meaning that the radius of an outer surface 672 is generally of a fixed value from a flow element 652 about the circumference of the flow element 652. Nasal prong 616 thus has a circular form factor when viewed from above, as is indicated generally in FIG. 11A. In contrast, nasal prong 716 has an elliptical form factor wherein an outer surface 772 varies in a generally elliptical fashion with respect flow element 752 about the circumference flow element 752. Nasal prong 716 thus appears elliptical or oblong when viewed from above, as is depicted generally in FIG. 11B. By way of further example, nasal prong 816 depicts an outer surface 872 that is of a completely different shape than either of the outer surfaces 672 and 772, and thus has a completely different form factor than either of nasal prongs 617 and 716. In this regard, it thus can be understood that nasal prong 816 may represent a custom-designed form factor having a radius that varies in, perhaps, an asymmetrical fashion between the outer surface 872 the flow element 852 about the circumference of nasal prong 816, and that is may be configured for a specific patient or may be configured for a specific class of patients.

By way of further example, FIGS. 12A and 12B depict that in other embodiments the nasal prong need not have its flow element situated at its mass or area centroid. For instance, FIG. 12A depicts nasal prong 616 and demonstrates with a set of indication lines 694 that the center of its flow element 652 and its centroid generally overlie one another. That is, the flow element 652 is situated generally at the center of mass and area of the seal apparatus of nasal prong 616. The same can be generally said of nasal prongs 716 and 816 as indicated in FIGS. 11B and 11C. However, FIG. 12B depicts a nasal prong 916 in accordance with a tenth embodiment that includes a flow element 952 that is offset from the center of mass of its seal apparatus. That is, FIG. 12B depicts at the numeral 994A a set of indication lines that represent the center of mass of a seal apparatus 936 and further depict with another set of indication lines 994B a center of a flow element 952. In particular, it can be seen that indication lines 994A and 994B are offset from one another. Such an offsetting can potentially provide an advantageously generous amount of material of the seal apparatus at a particular location within a nostril of a patient, such as may enable nasal prong 916 to be specially configured for a particular patient or for a particular class of patients.

It is to be understood that the various profiles of the nasal prongs 316, 416, and 516 and the various form factors of the nasal prongs 616, 716, and 816, and the potential offsetting of the flow element 952 from the center of mass of the seal apparatus 936 depicted generally in FIG. 12B can be implemented in any combination in conjunction with any of the seal apparatuses 36, 136, and 236 without limitation. The various embodiments depicted herein thus demonstrate that numerous configurations of nasal prongs can be configured to compressively engage an inner surface of a nostril to advantageously promote the provision of a flow of breathing gases to a patient.

In the claims, any reference signs placed between parentheses shall not be construed as limiting the claim. The word "comprising" or "including" does not exclude the presence of elements or steps other than those listed in a claim. In a device claim enumerating several means, several of these means may be embodied by one and the same item of hardware. The word "a" or "an" preceding an element does not exclude the presence of a plurality of such elements. In any device claim enumerating several means, several of these means may be embodied by one and the same item of hardware. The mere fact that certain elements are recited in mutually different dependent claims does not indicate that these elements cannot be used in combination.

Although the invention has been described in detail for the purpose of illustration based on what is currently considered to be the most practical and preferred embodiments, it is to be understood that such detail is solely for that purpose and that the invention is not limited to the disclosed embodiments, but, on the contrary, is intended to cover modifications and equivalent arrangements that are within the scope of the appended claims. For example, it is to be understood that the present invention contemplates that, to the extent possible, one or more features of any embodiment can be combined with one or more features of any other embodiment.

## Claims

1. A nasal prong (216) structured to be situated on a nasal cushion (20) of a patient interface device the nasal prong (216) comprising:
a resilient seal apparatus (236) which comprises a deformable element (264) and a flexible enclosure (268) that possesses elastic and resilient properties, at least a portion of the deformable element (264) being situated within the flexible enclosure (268), wherein the resilient seal apparatus (236) is structured to be deformable between a free state and a deformed state from among a plurality of deformed states in response to a force applied to the seal apparatus (236) and being further structured to return from the deformed state in a direction generally toward the free state upon removal of the force, at least a portion of the seal apparatus (236) that is in response to compressive forces applied to the seal apparatus (236) in a first deformed state and that is received in a nostril of the patient being structured to move toward the free state and to compressively and sealingly engage the nostril in a second deformed state that is generally between the first deformed state and the free state; and
a flow channel (256) extending through the seal apparatus (236), the flow channel (52) being structured to be in fluid communication with the source of breathing gas and to provide the flow of breathing gas into the nostril,
**characterized in that** the deformable element (264) comprises a quantity of fluid in a liquid state and being retained within the enclosure (268), the enclosure (268) including a first portion (284) and a second portion (286) that are formed out of the same material, wherein the first portion (284) has a relatively greater stiffness in bending than the second portion (286).

2. The nasal prong of claim 1, wherein the enclosure (268) is elastically deflectable from its free state in conjunction with deformation of at least a portion of the enclosure (268) and the quantity of fluid into the first deformed state.

3. The nasal prong of claim 2, wherein the first portion (284) and the second portion (286) are connected together and disposed in contact with the quantity of fluid, the enclosure (268) being at least one of the first portion and the second portion.

4. The nasal prong of claim 3, wherein one of the first portion (284) and the second portion (286) is structured to be situated proximal to the nasal cushion (20) and the other of the first portion (284) and the second portion (286) is structured to be situated distal the nasal cushion (20), the enclosure (268) being the one of the first portion (284) and the second portion (286).

5. The nasal prong of claim 4, wherein:
the flow channel (256) comprises an elongated flow element (252) having an opening formed therein that extends throughout the longitudinal extent of the flow element (252) and that is structured to be in fluid communication with the source of breathing gas;
the first portion (284) has a connection (288) with the flow element (252);
the second portion (286) has a connection (290) with the flow element (252);
the first and second portions (284, 286) are connected together at another connection (292) opposite the connections with the flow element (252); and
the quantity of fluid is enclosed among the first portion (284), the second portion (286), and the flow element (252).

6. The nasal prong of claim 1, wherein the first portion (284) has a relatively greater thickness (285) than that (287) of the second portion (286).

7. A patient interface device (24) comprising:
a cushion (20); and
a pair of the nasal prongs (216) according to one of claims 1 to 6 operatively coupled to the cushion (20).

## Patentansprüche

1. Nasengabel (216), die dazu ausgebildet ist, auf einem Nasenpolster (20) einer Patientenschnittstellenvorrichtung zu liegen, wobei die Nasengabel (216) umfasst:
eine nachgiebige Dichtungseinrichtung (236), die ein verformbares Element (264) und eine flexible Kapsel (268) umfasst, die elastische und nachgiebige Eigenschaften besitzt, wobei wenigstens ein Abschnitt des verformbaren Elements (264) innerhalb der flexiblen Kapsel (268) liegt, wobei die nachgiebige Dichtungseinrichtung (236) dazu ausgebildet ist, in Reaktion auf eine Kraft, die an die Dichtungseinrichtung (236) angelegt wird, zwischen einem freien Zustand und einem verformten Zustand aus einer Vielzahl von verformten Zuständen verformbar zu sein, und weiter dazu ausgebildet ist, bei Aufheben der Kraft aus dem verformten Zustand in eine Richtung allgemein zu dem freien Zustand hin zurückzukehren, wobei wenigstens ein Abschnitt der Dichtungseinrichtung (236), der sich in Reaktion auf an die Dichtungseinrichtung (236) angelegte Kompressionskräfte in einem ersten verformten Zustand befindet und der in einem Nasenloch des Patienten aufgenommen ist, dazu ausgebildet ist, sich zu dem freien Zustand hin zu bewegen und das Nasenloch in einem zweiten verformten Zustand, der sich allgemein zwischen dem ersten verformten Zustand und dem freien Zustand befindet, kompressiv und abdichtend in Eingriff zu nehmen; und
einen Strömungskanal (256), der sich durch die Dichtungseinrichtung (236) erstreckt, wobei der Strömungskanal (52) dazu ausgebildet ist, sich mit der Atemgasquelle in Fluidkommunikation zu befinden und den Atemgasstrom in das Nasenloch bereitzustellen,
**dadurch gekennzeichnet, dass** das verformbare Element (264) eine Menge an Fluid umfasst, das sich in einem flüssigen Zustand befindet und innerhalb der Kapsel (268) gehalten wird, wobei die Kapsel (268) einen ersten Abschnitt (284) und einen zweiten Abschnitt (286) einschließt, die aus dem gleichen Material gebildet sind, wobei der erste Abschnitt (284) eine relativ höhere Biegesteifigkeit besitzt als der zweite Abschnitt (286).

2. Nasengabel nach Anspruch 1, wobei die Kapsel (268) in Verbindung mit Verformung von wenigstens einem Abschnitt der Kapsel (268) und der Fluidmenge elastisch aus ihrem freien Zustand in den ersten verformten Zustand auslenkbar ist.

3. Nasengabel nach Anspruch 2, wobei der erste Abschnitt (284) und der zweite Abschnitt (286) miteinander verbunden und in Kontakt mit der Fluidmenge angeordnet sind, wobei die Kapsel (268) wenigstens eines aus dem ersten Abschnitt und dem zweiten Abschnitt ist.

4. Nasengabel nach Anspruch 3, wobei einer aus dem ersten Abschnitt (284) und dem zweiten Abschnitt (286) dazu ausgebildet ist, proximal zu dem Nasenpolster (20) zu liegen und der andere aus dem ersten Abschnitt (284) und dem zweiten Abschnitt (286) dazu ausgebildet ist, distal des Nasenpolsters (20) zu liegen, wobei die Kapsel (268) der eine aus dem ersten Abschnitt (284) und dem zweiten Abschnitt (286) ist.

5. Nasengabel nach Anspruch 4, wobei:
der Strömungskanal (256) ein längliches Strömungselement (252) umfasst, das eine darin gebildete Öffnung besitzt, die sich durch die Längserstreckung des Strömungselements (252) erstreckt und die dazu ausgebildet ist, sich mit der Atemgasquelle in Fluidkommunikation zu befinden;
der erste Abschnitt (284) eine Verbindung (288) mit dem Strömungselement (252) besitzt;
der zweite Abschnitt (286) eine Verbindung (290) mit dem Strömungselement (252) besitzt;
der erste und zweite Abschnitt (284, 286) an einer anderen Verbindung (292) gegenüber den Verbindungen mit dem Strömungselement (252) miteinander verbunden sind; und
die Fluidmenge zwischen dem ersten Abschnitt (284), dem zweiten Abschnitt (286), und dem Strömungselement (252) eingekapselt ist.

6. Nasengabel nach Anspruch 1, wobei der erste Abschnitt (284) eine relativ größere Dicke (285) als die (287) des zweiten Abschnitts (286) besitzt.

7. Patientenschnittstellenvorrichtung (24), umfassend:
ein Polster (20); und
ein Nasengabelpaar (216) nach einem der Ansprüche 1 bis 6, das betriebsmäßig mit dem Polster (20) gekoppelt ist.

## Revendications

1. Pince nasale (216) structurée pour être située sur un coussinet nasal (20) d'un dispositif d'interface patient, la pince nasale (216) comprenant :
un dispositif d'étanchéité résilient (236) qui comprend un élément déformable (264) et une enveloppe flexible (268) qui possède des propriétés élastiques et résilientes, au moins une partie de l'élément déformable (264) étant située dans l'enveloppe flexible (268), dans laquelle le dispositif d'étanchéité résilient (236) est structuré pour être déformable entre un état libre et un état déformé, à partir d'une pluralité d'états déformés, en réponse à une force appliquée au dispositif d'étanchéité (236) et étant en outre structurée pour revenir de l'état déformé dans une direction généralement vers l'état libre, lors de la suppression de la force, au moins une partie du dispositif d'étanchéité (236), qui est en réponse à des forces de compression appliquées au dispositif d'étanchéité (236), dans un premier état déformé et qui est reçue dans une narine du patient étant structurée pour se déplacer vers l'état libre et pour mettre en prise de manière compressive et étanche la narine dans un second état déformé, qui est généralement entre le premier état déformé et l'état libre ; et
un canal d'écoulement (256) s'étendant à travers le dispositif d'étanchéité (236), le canal d'écoulement (52) étant structuré pour être en communication fluidique avec la source de gaz respiratoire et pour permettre l'écoulement de gaz respiratoire dans la narine,
**caractérisée en ce que** l'élément déformable (264) comprend une quantité de fluide dans un état liquide et retenu dans l'enveloppe (268), l'enveloppe (268) comprenant une première partie (284) et une seconde partie (286), qui sont formées dans le même matériau, dans laquelle la première partie (284) a une rigidité à la flexion relativement supérieure à la seconde partie (286).

2. Pince nasale selon la revendication 1, dans laquelle l'enveloppe (268) peut être élastiquement défléchie, depuis son état libre en combinaison avec la déformation d'au moins une partie de l'enveloppe (268) et la quantité de fluide dans le premier état déformé.

3. Pince nasale selon la revendication 2, dans laquelle la première partie (284) et la seconde partie (286) sont connectées ensemble et disposées en contact avec la quantité de fluide, l'enveloppe (268) étant au moins une parmi la première partie et la seconde partie.

4. Pince nasale selon la revendication 3, dans laquelle une de la première partie (284) et la seconde partie (286) est structurée pour être située de manière proximale par rapport au coussinet nasal (20) et l'autre de la première partie (284) et de la seconde partie (286) est structurée pour être située de manière distale par rapport au coussinet nasal (20), l'enveloppe (268) étant l'une de la première partie (284) et la seconde partie (286).

5. Pince nasale selon la revendication 4, dans laquelle :
le canal d'écoulement (256) comprend un élément d'écoulement allongé (252) ayant une ouverture formée à l'intérieur qui s'étend à travers l'amplitude longitudinale de l'élément d'écoulement (252) et qui est structurée pour être en communication fluidique avec la source du gaz respiratoire ;
la première partie (284) a une connexion (288) avec l'élément d'écoulement (252) ;
la seconde partie (286) a une connexion (290) avec l'élément d'écoulement (252) ;
la première et la seconde parties (284, 286) sont connectées ensemble à une autre connexion (292) opposée aux connexions avec l'élément d'écoulement (252) ; et
la quantité de fluide est contenue parmi la première partie (284), la seconde partie (286) et l'élément d'écoulement (252).

6. Pince nasale selon la revendication 1, dans laquelle la première partie (284) a une épaisseur relativement supérieure (285) à celle (287) de la seconde partie (286).

7. Dispositif d'interface patient (24) comprenant :
un coussinet (20) ; et
une paire de pinces nasales (216) selon l'une des revendications 1 à 6, opérationnellement raccordées au coussinet (20).
